# EUROPEAN PATENT SPECIFICATION

(11) **EP 1 665 976 B1**
(45) Date of publication and mention of the grant of the patent: **17.07.2013**
(21) Application number: 04772973.6
(22) Date of filing: 07.09.2004
(51) Int. Cl.: A61B 1/00

(54) **IN-SUBJECT INTRODUCING DEVICE AND WIRELESS IN-SUBJECT INFORMATION CAPTURING SYSTEM**
VORRICHTUNG ZUR EINFÜHRUNG IN PERSONEN UND DRAHTLOSES INFORMATIONSERFASSUNGSSYSTEM IN EINER PERSON
DISPOSITIF D'INTRODUCTION DANS UN SUJET, ET SYSTEME DE SAISIE D'INFORMATION SANS FIL A L'INTERIEUR DU SUJET

(30) Priority: 08.09.2003 JP 2003315542
(43) Date of publication of application: 07.06.2006
(73) Proprietor: Olympus Corporation, Tokyo 151-0072 (JP)
(72) Inventor: HASHIMOTO, Masayuki, Shibuya-ku, Tokyo 151-0072 (JP); NAKATSUCHI, Kazutaka, Hino-shi, Tokyo 1910062 (JP); FUJIMORI, Noriyuki, Suwa-shi, Nagano 3920131 (JP)
(74) Representative: von Hellfeld, Axel
(86) International application number: PCT/JP2004/013291
(87) International publication number: WO 2005/023102

(56) References cited:
- WO-A-01/87377
- WO-A-2004/054430
- JP-A- 2002 186 672
- JP-A- 2003 038 424
- JP-A- 2003 070 728
- US-A1- 2003 020 810

## Description

### TECHNICAL FIELD

The present invention relates to a subject introduction device that is used in a state where the device is introduced into a subject and executes predetermined functions in the subject, and a wireless in vivo information acquiring system using the subject introduction device.

### BACKGROUND ART

Swallowing capsule endoscopes are known in the fields of endoscopes. The capsule endoscopes have an imaging function and a wireless communication function. After a capsule endoscope is swallowed through a mouth of a patient for observation (examination) until it comes out of a body naturally, the capsule endoscope moves in a body cavity including internal organs such as stomach and small intestine according to the peristaltic movement so as to successively capture their images.

Image data captured in a body by the capsule endoscope, while moving in the body cavity, are successively transmitted to an outside through wireless communication, and are stored in a memory of an external receiver. When a patient carries the receiver having the wireless communication function and the memory function, the patient can move freely even after the patient swallows the capsule endoscope and before the capsule comes out of the body. Thereafter, doctors or caretakers make a display device display images of organs based on the image data stored in the memory so as to be capable of making a diagnosis.

While a driving power of such capsule endoscopes may be fed from a built-in power supply, in recent years an attention is paid to a configuration in which the driving power is fed from the outside via wireless transmission to the capsule endoscopes. Such a configuration in which the power is fed from the outside can avoid whole power from being consumed accidentally and the driving from being stopped while the capsule endoscope moves in a body cavity.

To control the driving of the capsule endoscope, the configuration, in which a reed switch for turning on/off the capsule endoscope according to an external magnetic field is provided into the capsule endoscope and a permanent magnet for applying a magnetic field is provided to a package that houses the capsule endoscope, is suggested. The reed switch provided into the capsule endoscope maintains an OFF state under an environment that the external magnetic field of certain strength or more is applied, and turns into an ON state when the strength of the external magnetic field is decreased. Therefore, the capsule endoscope is not driven in the state where it is housed in the package, whereas the capsule endoscope, when taken out of the package, is out of the influence of the permanent magnet and starts driving. Such a configuration can prevent the capsule endoscope from starting to be driven while they are housed in the package (for example, see Published International Application WO 01/35813).

Even when the mechanism that controls the driving state of the capsule endoscope is provided, however, the driving of the capsule endoscope that is outside a subject cannot be always prevented. In other words, since it takes certain time from when the capsule endoscope is taken out from the package to when the capsule endoscope is introduced into the subject, the capsule endoscope starts driving before it is introduced into the subject. A problem, which arises when the capsule endoscope starts driving before it is introduced into the subject, is explained below.

When the capsule endoscope starts driving before it is introduced into the subject, useless image data not used for diagnoses are obtained. Since the capsule endoscope is configured to start driving and capturing an image, and to wirelessly transmit the image data obtained, when the capsule endoscope is driven before it is introduced into the subject, operations including the image capturing are performed on the outside of the subject.

As a result, a lot of image data are obtained while the capsule is unsealed and then the capsule endoscope is introduced into the subject, and thus the doctors or the like make diagnoses after the useless image data are deleted. Since an imaging rate of the general capsule endoscope is about two images per second, when the capsule endoscope is driven on the outside of the subject, a lot of unnecessary image data are obtained even for a short time of about a few dozen of seconds. To avoid obtaining such useless image data, therefore, it is necessary to prevent the capsule endoscope from starting driving before the capsule endoscope is introduced into the subject.

Since a certain amount of the driving power is required for obtaining such useless image data, when the capsule endoscope is driven outside the subject, the electric power stored in the capsule endoscope is consumed away. Also from a viewpoint of the power consumption, therefore, it is necessary to prevent the capsule endoscope from starting driving before it is introduced into the subject.

It is necessary to check an operation of the capsule endoscope before it is taken orally, in which case it is desired to consume the bare minimum of electric power and suppress radiation of an unnecessary radio wave.

In view of the foregoing, an object of the present invention is to provide a subject introduction device and a wireless in vivo information acquiring system capable of an efficient and reliable capturing of an image at a desirable position and a suppression of an undesirable radiation of radio wave, while suppressing power consumption after the introduction of the subject introduction device into the subject.
WO 2004/054430 A2 was not public at the priority date of the present patent application. Such document discloses various embodiments of capsule endoscopes and, in particular a capsule endoscope with a pH tester or a thermometer, a pressure sensor or a blood sensor.
US 2003/0020810 A1 discloses some embodiments of capsule endoscopes. A sensor is provided for detecting that the capsule endoscope is in the colon so as to start imaging therefrom. The sensor can be a pH sensor, a speed sensor, an acceleration sensor or a pressure sensor. According to two embodiments, the endoscope is coated with a melting cover, consisting in an azo-polymer film. The azo-polymer film melts only in the colon because it is only melted by a specific enzyme generated by bacteria in the colon.

### DISCLOSURE OF THE INVENTION

In order to solve the problems as described above and to achieve an object, a subject introduction device used in a subject to execute predetermined functions in the subject includes: a function executing unit that executes the predetermined functions; a humidity sensor that detects that the subject introduction device is located in the subject via an opening provided in an outer surface of a package of the subject introduction device; a cover that covers the opening with a covering material that reacts with a substance in the subject when the subject introduction device comes into contact with the substance located along a path of the subject introduction device, and that exposes the opening with an advance of the reaction over time; and a driving controller that drive controls the function executing unit when the sensor detects that the subject introduction device is located in the subject. The cover is formed with a material that melts in the substance in the subject, said substance being water or a suitable predisposing material to be introduced in the stomach.

Still further, in the subject introduction device as described above, the cover sets an exposure time period of the opening according to a thickness of the covering material at the opening.

Still further, in the subject introduction device as described above, the sensor detects predetermined characteristics of the substance in the subject.

Still further, in the subject introduction device as described above, the function executing unit is a wireless transmitter that includes a wireless actuating switch that actuates the wireless transmitter, and the driving controller turns on the wireless actuating switch when the sensor detects that the subject introduction device is located in the subject.

Still further, the subject introduction device as described above includes a timer that counts a predetermined time after the sensor detects that the subject introduction device is located in the subject, and switches the wireless actuating switch on when the timer counts up the predetermined time.

Still further, in the subject introduction device as described above, the function executing unit is a wireless transmitter that includes a wireless power source changing unit that switches between a low power consumption mode and a normal power consumption mode of the wireless transmitter, and the driving controller sends a change direction to the wireless power source changing unit to switch from the low power consumption mode to the normal power consumption mode when the sensor detects that the subject introduction device is located in the subject or when the timer counts up the predetermined time.

Still further, in the subject introduction device as described above, the timer is provided into an imaging circuit.

Still further, the subject introduction device as described above further includes a frame rate changing unit which changes between a first imaging process at a predetermined frame rate and a second imaging process at a frame rate higher than the predetermined frame rate, and the driving controller sends a direction to the frame rate changing unit to perform image capturing at the frame rate of the first imaging process after the power source switch is on, and sends a direction to perform image capturing at the frame rate of the second imaging process when the body determining unit determines that the subject introduction device is located in the subject or the timer counts up to the predetermined time.

Still further, a wireless in vivo information acquiring system includes: a subject introduction device as defined in claim 1 to be introduced into a subject; and a receiver which is arranged out of the subject, and receives information obtained by the subject introduction device, via wireless communication. The receiver includes a wireless receiver which receives the information transmitted from the wireless transmitter; and a processor which analyzes the information received.

Still further, in the wireless in vivo information acquiring system as described above, the driving controller turns on a wireless switch that actuates the wireless transmitter when the sensor detects that the subject introduction device is located in the subject.

### BRIEF DESCRIPTION OF THE DRAWINGS

Fig. 1 is a schematic diagram of a wireless in vivo information acquiring system according to a first embodiment of the present invention;
Fig. 2 is a block diagram of a transmitting/receiving device of the in vivo information acquiring system shown in Fig. 1;
Fig. 3 is a block diagram of a capsule endoscope of the in vivo information acquiring system shown in Fig. 1;
Fig. 4 is a flowchart of a driving control procedure in the capsule endoscope by a driving controller;
Fig. 5 is a block diagram of a modification of the capsule endoscope according to the first embodiment;
Fig. 6 is a block diagram of another modification of the capsule endoscope according to the first embodiment;
Fig. 7 is a block diagram of a capsule endoscope according to a second embodiment of the present invention; and
Fig. 8 is a flowchart of a driving control procedure in the capsule endoscope by a driving controller shown in Fig. 7.

### BEST MODE FOR CARRYING OUT THE INVENTION

Exemplary embodiments of the present invention will be described in detail hereinbelow, though the invention is not limited thereby.

### FIRST EMBODIMENT

First, a wireless in vivo information acquiring system according to a first embodiment is explained. The wireless in vivo information acquiring system according to the first embodiment employs a capsule endoscope as a subject introduction device.

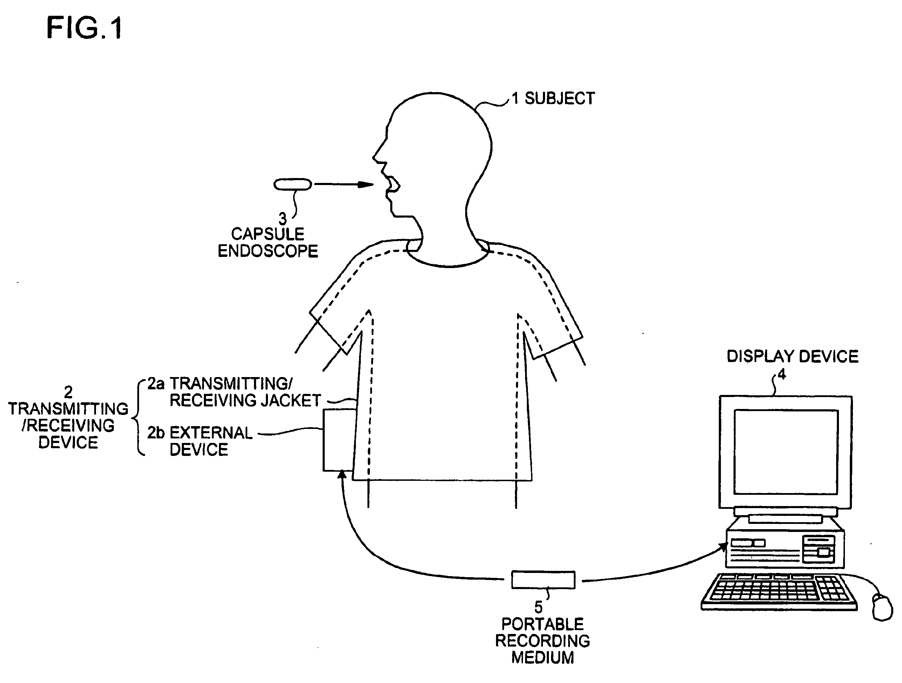

Fig. 1 is a schematic diagram of the wireless in vivo information acquiring system according to the first embodiment. As shown in Fig. 1, the wireless in vivo information acquiring system has a transmitting/receiving device 2 having a wireless transmitting/receiving function, and a capsule endoscope (subject introduction device) 3 that is introduced into a subject 1, is operated by driving power obtained from a radio signal transmitted from the transmitting/receiving device 2, and captures images in a body cavity so as to transmit data to the transmitting/receiving device 2. Further, the wireless in vivo information acquiring system has a display device 4 that displays images in the body cavity based on the data received by the transmitting/receiving device 2, and a portable recording medium 5 that transmits/receives the data between the transmitting/receiving device 2 and the display device 4. The transmitting/receiving device 2 has a transmitting/receiving jacket 2a worn by the subject 1, and an external device 2b that processes a radio signal transmitted/received via the transmitting/receiving jacket 2a.

The display device 4 is for displaying the images in the body cavity captured by the capsule endoscope 3, and has a configuration of a work station or the like for displaying the images based on data obtained from the portable recording medium 5. Specifically, the display device 4 may have a configuration such that the images are displayed directly on a CRT display, a liquid crystal display or the like, or a configuration such that the images are output to another medium such as a printer.

The portable recording medium 5 is detachable from the external device 2b and the display device 4, and has a configuration such that when inserted to be attached to one of them, information can be output or recorded. Specifically, the portable recording medium 5 is inserted to be attached into the external device 2b so as to record data transmitted from the capsule endoscope 3 while the capsule endoscope 3 is moving in the body cavity of the subject 1. After the capsule endoscope 3 comes out from the subject 1, namely, after the imaging of the inside of the subject 1 is completed, the portable recording medium 5 is taken out of the external device 2b and is inserted to be attached into the display device 4, and the recorded data are read by the display device 4. The data are transmitted/received between the external device 2b and the display device 4 by the portable recording medium 5 such as a compact flash (registered trademark) memory, and hence, the subject 1 can move freely during the image capturing of the body cavity unlike the case where the external device 2b and the display device 4 are connected with each other by a wire.

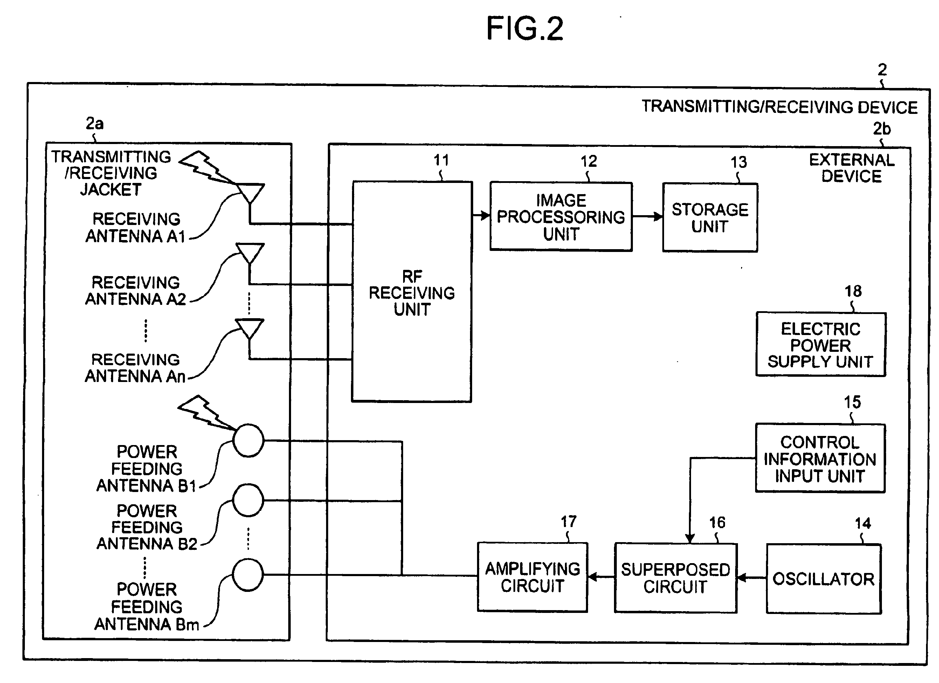

The transmitting/receiving device 2 has a function as a power feeding device for transmitting electric power to the capsule endoscope 3, and also a function as a receiving device that receives image data of the body cavity wirelessly transmitted from the capsule endoscope 3. Fig. 2 is a block diagram of the transmitting/receiving device 2. As shown in Fig. 2, the transmitting/receiving device 2 can be worn by the subject 1, and has the transmitting/receiving jacket 2a having receiving antennas A1 to An and power feeding antennas B1 to Bm, and the external device 2b that processes a transmitted/received radio signal.

The external device 2b has a function for processing the radio signal transmitted from the capsule endoscope 3. Specifically, as shown in Fig. 2, the external device 2b has an RF receiver 11 that executes a predetermined process such as decoding a radio signal received by the receiving antennas A1 to An and extracts image data obtained by the capsule endoscope 3 from the radio signal so as to output the image data, an image processor 12 that executes a process necessary for the output image data, and a storage unit 13 that records the image data after the image process. The image data are recorded in the portable recording medium 5 via the storage unit 13.

The external device 2b has a function for generating a radio signal transmitted to the capsule endoscope 3. Specifically, the external device 2b has an oscillator 14 that generates a power feeding signal and defines an oscillation frequency, a control information input unit 15 that generates a control information signal for controlling a driving state of the capsule endoscope 3, a superposed circuit 16 that synthesizes the power feeding signal with the control information signal, and an amplifying circuit 17 that amplifies strength of the synthesized signal. The signal amplified by the amplifying circuit 17 is transmitted to the power feeding antennas B1 to Bm so as to be transmitted to the capsule endoscope 3. The external device 2b includes a power supply unit 18 having a predetermined capacitor, an AC power source adapter or the like, and the components of the external device 2b use electric power supplied from the power supply unit 18 as a driving energy.

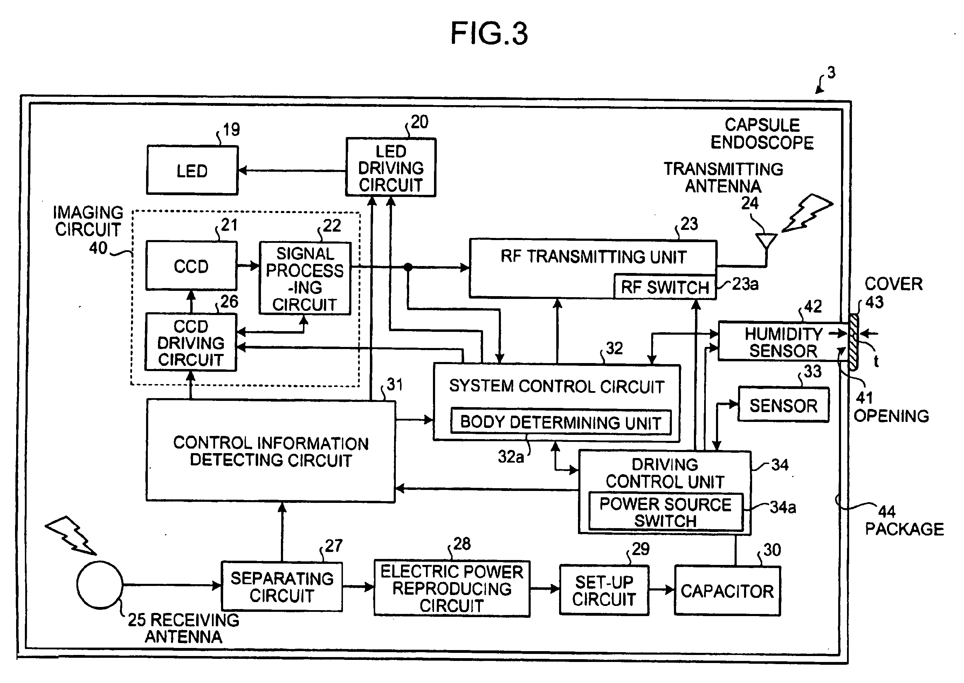

The capsule endoscope 3 is explained. Fig. 3 is a block diagram of a configuration of the capsule endoscope 3. As shown in Fig. 3, the capsule endoscope 3 has a light emitting diode (LED) 19 that irradiates an imaging area when the image inside the subject 1 is captured, an LED driving circuit 20 that controls a driving state of the LED 19, a charge coupled device (CCD) 21 that captures a reflected light image from the area irradiated by the LED 19, and a signal processing circuit 22 that processes an image signal output from the CCD 21 into imaging information having a desired format. Further, the capsule endoscope 3 has a CCD driving circuit 26 that controls a driving state of the CCD 21, an RF transmitter 23 that modulates the image data captured by the CCD 21 by the signal processing circuit 22 so as to generate an RF signal, a transmitting antenna 24 that wirelessly transmits the RF signal output from the RF transmitter 23, and a system control circuit 32 that controls operations of the LED driving circuit 20, the CCD driving circuit 26, and the RF transmitter 23. The CCD 21, the signal processing circuit 22, and the CCD driving circuit 26 are collectively called as an imaging circuit 40.

When these mechanisms are provided, while the capsule endoscope 3 is in the subject 1, image information about portions to be examined irradiated by the LED 19 is acquired by the CCD 21. The signal processing circuit 22 executes the signal process on the acquired image information, and after the RF transmitter 23 converts the image information into an RF signal, it transmits the RF signal to the outside via the transmitting antenna 24.

The capsule endoscope 3 has a receiving antenna 25 that receives a radio signal transmitted from the transmitting/receiving device 2, and a separating circuit 27 that separates a power feeding signal from the signal received by the receiving antenna 25. Further, the capsule endoscope 3 has an electric power reproducing circuit 28 that reproduces electric power from the separated power feeding signal, a set-up circuit 29 that sets up the reproduced electric power, and a capacitor 30 that stores the set-up electric power. Further, the capsule endoscope 3 has a control information detecting circuit 31 that detects contents of a control information signal from a component separated from the power feeding signal by the separating circuit 27, and outputs a control signal to the LED driving circuit 20, the CCD driving circuit 22, and the system control circuit 32 if necessary. The control information detecting circuit 31 and the system control circuit 32 also have a function for distributing the driving power supplied form the capacitor 30 to another component.

The capsule endoscope 3 having these components receives a radio signal transmitted from the transmitting/receiving device 2 via the receiving antenna 25, and separates a power feeding signal and a control information signal from the received radio signal. The control information signal is output to the LED driving circuit 20, the CCD driving circuit 22, and the system control circuit 32 via the control information detection circuit 31 so as to be used for controlling the driving states of the LED 19, the CCD 21, and the RF transmitter 23. On the other hand, the electric power reproducing circuit 28 reproduces the power feeding signal as electric power, and the set-up circuit 29 sets up the reproduced electric power to a potential of the capacitor 30 so that the set-up electric power is stored in the capacitor 30. The capacitor 30 has a configuration for being capable of supplying electric power to the system control circuit 32 and the other components. The capsule endoscope 3 is configured so that electric power is supplied by wireless transmission from the transmitting/receiving device 2.

The capsule endoscope 3 has a sensor 33 that detects predetermined magnetic, light and wave signals and the like, a humidity sensor 42, and a driving controller 34 that control driving states of various function executing units such as the system control circuit 32, the RF transmitter 23, and the imaging circuit 40. The driving controller 34 has a power source switch 34a as a main switch for a power source of the entire capsule endoscope 3. The sensor 33 detects magnetism, light, wave and the like as signals for an on/off operation of the power source switch 34a, so as to output a detected result to the driving controller 34. The RF transmitter 23 has an RF switch 23a as a power source switch for the entire RF transmitter 23. The system control circuit 32 has a body determining unit 32a, and the body determining unit 32a determines whether humidity is humidity in a subject based on a detected result from the humidity sensor 42 so as to determine whether the capsule endoscope 3 is present in the subject or on the outside of the subject. When the driving controller 34 acquires a determined result from the body determining unit 32a indicating that the capsule endoscope 3 is present in the subject, the RF switch 23a is turned into an ON state so as to actuate the RF transmitter 23.

The entire capsule endoscope 3 is covered with a package 44. The humidity sensor 42 is exposed from the capsule endoscope 3 via an opening 41 partially provided on the package 44. In a state, however, before the capsule endoscope 3 is used, a cover 43 is provided so as to externally cover the opening 41. The cover 43 is covered with a candy material. In the state before the capsule endoscope 3 is used, therefore, the humidity sensor 42 does not function. When the capsule endoscope 3 is introduced into the subject, the cover 43 as the candy material is gradually melted by saliva in the subject, and its thickness t becomes thinner. The cover 43 melts completely with time so that the opening 41 is exposed, and the humidity sensor 42 detects outer humidity. When the humidity sensor 42 detects the humidity in the subject, such as humidity (moisture) of the saliva and humidity (moisture) of gastric juice, the RF switch 23a is turned into the ON state so as to actuate the RF transmitter 23.

Since the opening 41 is not exposed to the outside as long as the capsule endoscope 3 is not introduced into a subject, the RF switch is not turned into the ON state outside the subject, and thus electric power is not consumed uselessly. Further, when the humidity determined by the body determining unit 32a is set to the humidity of saliva or the humidity of gastric juice, the RF switch can be operated into an ON state from a desired position in the subject. As a result, captured images in the desired position can be obtained with less consumption of power source capacity.

Instead of the humidity sensor 42, a pH sensor may be used in a non inventive variant. In this case, an ON time of the RF switch can be set according to a difference in pH of saliva and pH of gastric juice. The cover 43 is formed by the candy material, but it is not limited to this, and red food die or wafer may be used. Further, the cover 43 may be transparent. Further, the thickness of the cover 43 is set so that a time period for which the opening is exposed can be adjusted.

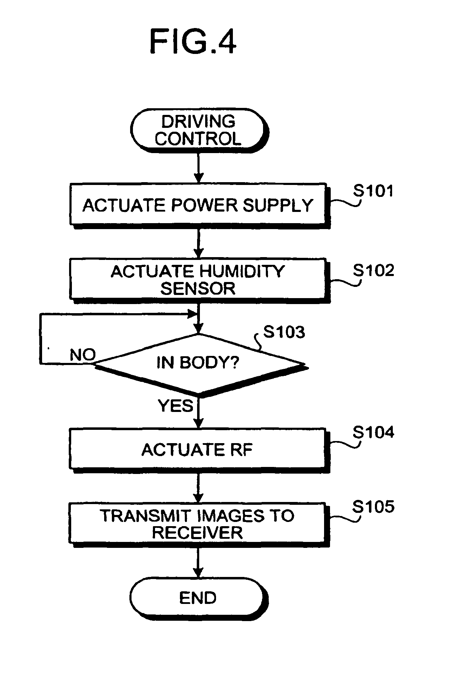

The driving control procedures of the respective units based on the determined result from the body determining unit 32a is explained with reference to Fig. 4. At the start time of the process, the components in the capsule endoscope 3 are in the OFF state. The sensor 33 does not require a power source, and detects a mechanical movement so as to bring the power source switch 34a into the ON/OFF state.

When the power source switch 34a is actuated (step S101), the driving controller 34 actuates at least the humidity sensor 43 (step S102). In this case, since the RF transmitter 23 is not actuated, even if the imaging circuit 40 is actuated, images obtained by the imaging circuit 40 are not transmitted to the outside.

Thereafter, the driving controller 34 determines whether a determined result such that the humidity sensor 42 is in a subject is gotten from the body determining unit 32a (step S103). When, for example, the humidity detected by the humidity sensor 42 is set as the humidity of saliva, it is determined that the humidity sensor 42 is in the subject when the humidity of saliva is detected. When not determined that the body determining unit 32a is in the subject (No at step S103), the driving controller 34 repeats the determining process at step S103.

On the other hand, when the body determining unit 32a determines that the capsule endoscope 3 is in the subject (Yes at step S103), it brings the RF switch 23a of the RF transmitter 23 into the ON state so as to actuate the RF transmitter 23 (step S104). When the imaging circuit 40 is not actuated then, it is actuated. After the RF transmitter 23 is actuated (step S104), image data imaged by the imaging circuit 40 are transmitted to the external device 2b via the RF transmitter 23 and the transmitting antenna 24 (step S105) so that the process is ended. A reception mechanism provided to the transmitting/receiving jacket 2a receives the transmitted image data, and supplies them to the display device 4 via the portable recording medium 5, and the image data are displayed as subject images on a screen of the display device 4.

In the first embodiment, when the body determining unit 32a determines that the capsule endoscope 3 is in the subject, it brings the RF switch 23 in the OFF state into the ON state, so that captured images are transmitted. When the power source switch 34a is brought into the ON state, however, it may actuate the RF transmitter 23 in a low-power consumption mode, and when the body determining unit 32a determines that the capsule endoscope 3 is in the subject, it may change the RF transmitter 23 into a normal power consumption mode.

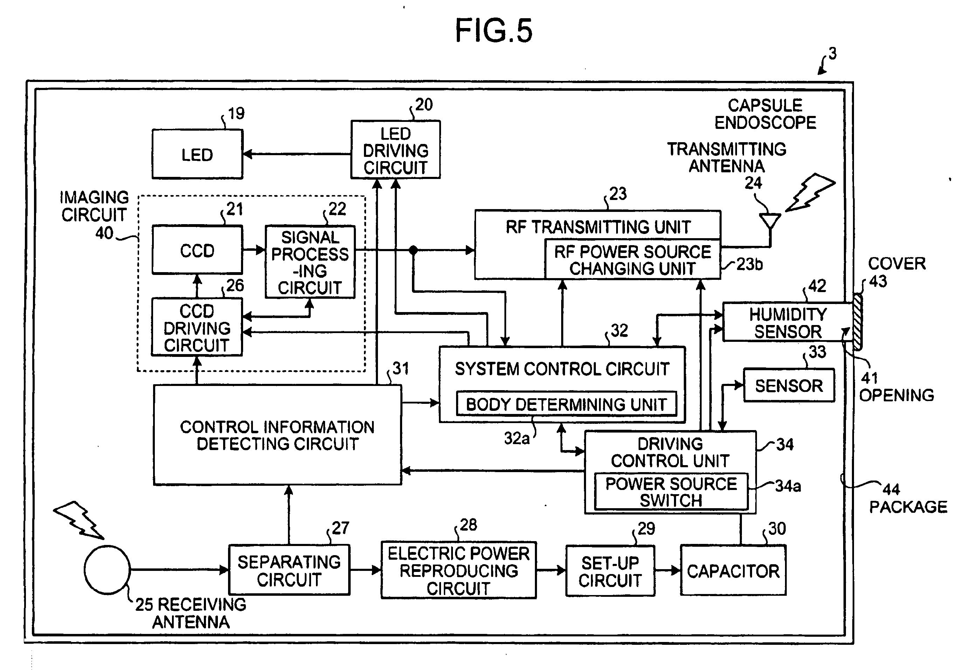

In this case, as shown in Fig. 5, the RF transmitter 23 has an RF power source changing unit 23b instead of the RF switch 23a. When the body determining unit 32a determines that the capsule endoscope 103 is out of the subject, the RF power source changing unit 23b sets the RF transmitter 23 into the low power consumption mode where it is driven by a lower power consumption than that in the normal state according to an instruction from the driving controller 34. When the body determining unit 32a determines that the capsule endoscope 103 is in the subject, the RF power source changing unit 23b changes the RF transmitter 23 into the normal power consumption mode where it is driven by the normal power consumption. As a result, a weak radio wave is transmitted from the transmitting antenna 24 in the low power consumption mode, and a radio wave having strength that can be received on the outside of the subject is output from the transmitting antenna 24 in the normal power consumption mode. When the capsule endoscope 103 is out of the subject, its operation is occasionally checked, and in this case, imaged data can be gotten by transmitting the weak radio wave. Therefore, the operation of the imaging circuit 40 can be checked.

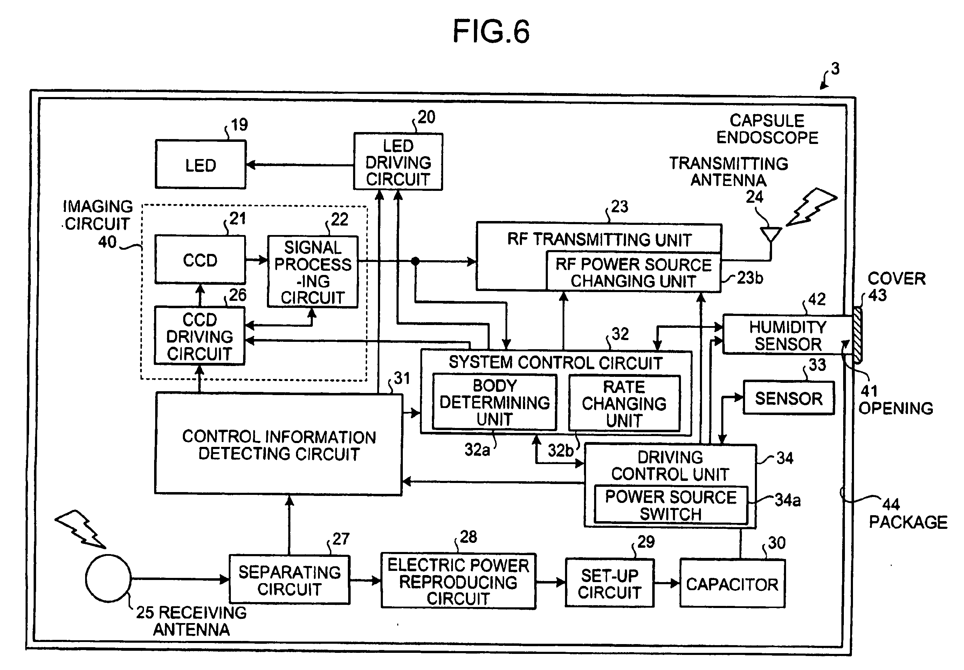

As shown in Fig. 6, a rate changing unit 32b may be provided, and when the body determining unit 32a determines that the capsule endoscope 203 is out of the subject, the rate changing unit 32b decreases an imaging frame rate of the imaging circuit 40. When the body determining unit 32a determines that the capsule endoscope 203 is in the subject, the rate changing unit 32b changes the imaging frame rate of the imaging circuit 40 into a normal rate. In Fig. 6, the RF power source changing unit 23b is used to further suppress radio wave radiation, thereby suppressing the power consumption. The power consumption can be, however, suppressed only by reducing the imaging frame rate.

In the first embodiment, with the provision of the cover 43, when the capsule endoscope 3 is introduced into the subject securely, the humidity sensor 42 is operated, so that useless power consumption is prevented when the capsule endoscope 3 is placed out of the subject. Further, the provision of the humidity sensor 42 enables desired images of desired positions in the subject imaged and transmitted.

### SECOND EMBODIMENT

A second embodiment of the present invention is explained below. In the first embodiment, the function of the humidity sensor 42 brings the components such as the RF switch 23a that consume a lot of power consumption into the operating state. In the second embodiment, however, the RF switch and the like can also be brought into the ON state in a desired position finely determined.

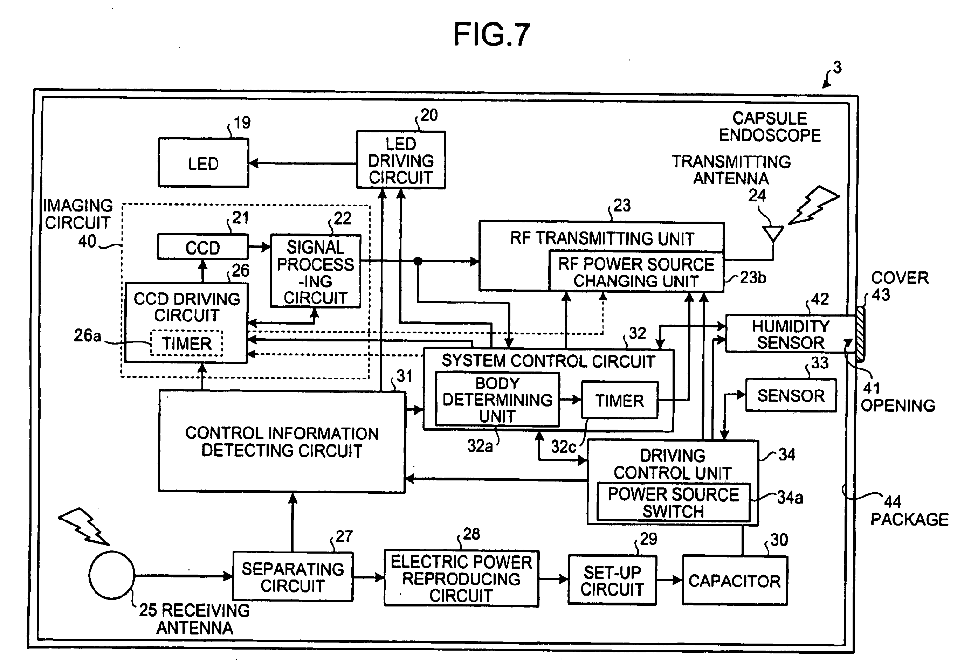

Fig. 7 is a block diagram of a configuration of the capsule endoscope according to the second embodiment. In the capsule endoscope 3 shown in Fig. 7, in addition to the configuration of the capsule endoscope 3 shown in Fig. 3, a timer 32c is provided. When the body determining unit 32a determines that the capsule endoscope 3 is in a subject, the timer 32c starts to count predetermined time so as to actuate the RF switch 23a when the predetermined time comes. In this case, the body determining unit 32a can determine that the position of the capsule endoscope 3 is in the subject, but the position is basically limited to an area such as a stomach where characteristic images can be gotten. It is occasionally desired that the data of the gotten Images is transmitted from a middle position of the area. In this occasion, the RF transmitter 23 can be brought into the normal transmission state from a finely desired position, and the data of the gotten images can be transmitted to the external device 2b. The timer 32c is provided to the system control circuit 32, but the position is not limited to this, it can be provided into the CCD driving circuit 26 having a timing generator or the like so that the configuration of the CCD driving circuit 26 is used effectively.

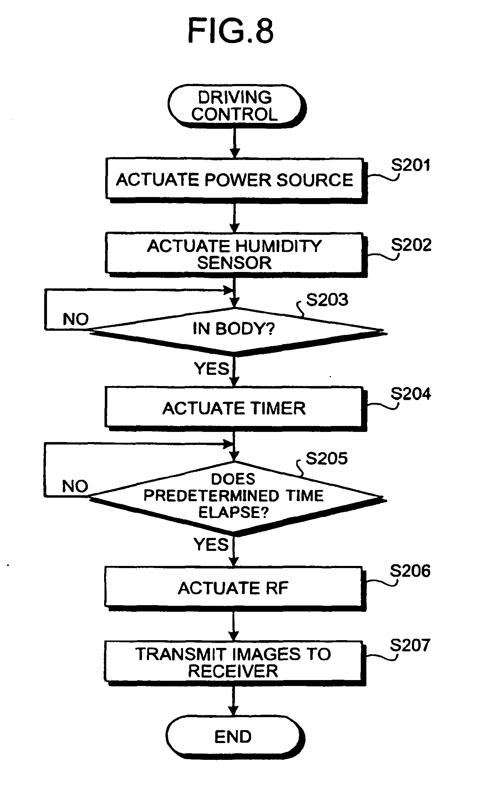

The driving control procedure of the respective units based on the determined result of the body determining unit 32a is explained with reference to a flowchart shown in Fig. 8. At the start time of this process, the components of the capsule endoscope 3 are in the OFF state. The sensor 33 does not require a power source, and detects, for example, a mechanical movement so as to bring the power source switch 34a into the ON/OFF state.

When the power source switch 34a is actuated (step S201), the driving controller 34 actuates at least the humidity sensor 42 (step S202). Thereafter, the driving controller 34 determines whether the determined result that the humidity sensor 42 is in a subject is gotten from the body determining unit 32a (step S203). When, for example, the humidity determined by the humidity sensor 42 is set to the humidity of saliva, it is determined that the humidity sensor 42 is in the subject when the humidity of saliva is detected. When the body determining unit 32a does not determine that the humidity sensor 42 is in the subject (No at step S203), the driving controller 34 repeats the determining process at step S103.

On the other hand, when the body determining unit 32a determines that the capsule endoscope 3 is in the subject (Yes at step S203), it actuates the timer 32c (step S204). Thereafter, the time counted by the timer 32c runs beyond the predetermined time (step S205). When the predetermined time does not elapse (No at step S205), the determining process at step S205 is repeated. On the other hand, when the predetermined time elapses (YES at step S205), the RF switch 23a of the RF transmitter 23 is brought into the ON state, so as to actuate the RF transmitter 23 (step S206). When the imaging circuit 40 is not actuated then, it is actuated. After the RF transmitter 23 is actuated, the image data imaged by the imaging circuit 40 are transmitted to the external device 2b via the RF transmitter 23 and the transmitting antenna 24 (step S207), and the process is ended. The receiving mechanism provided to the transmitting/receiving jacket 2a receives the transmitted image data, and supplies them to the display device 4 via the portable recording medium 5 so that the image data are displayed as the images of the inside of the subject on the screen of the display device 4.

In the second embodiment, in addition to the effects of the first embodiment, the RF switch 23a or the like can be actuated from a position of the subject finely determined, so that minimum necessary images can be gotten and the power consumption can be reduced to a minimum necessary level.

In the first and the second embodiments, the cover 43 is formed by, for example, a candy material that reacts to a substance in a path of the subject, through which the capsule endoscope 3 passes and which contacts with the capsule endoscope 3. The coating material is not limited to this, and a coating material that melts in water can be used when the substance in the subject is water used via the oral route. Further, a coating material that reacts to a predisposing material that is introduced into a stomach in advance can be used. In this case, the cover 43 melts in the stomach so that the opening 41 is exposed.

### INDUSTRIAL APPLICABILITY

As can be seen from the foregoing, the present invention is suitable for a subject introduction device and a wireless in vivo information acquiring system that allow the suppression of power consumption after the introduction of the subject introduction device into the subject, as well as the efficient and reliable image pick-up of a desirable image, while suppressing an undesirable radiation of electric waves.

## Claims

1. A subject introduction device (3) for insertion in a subject (1) to execute predetermined functions in the subject (1), comprising:
a function executing unit (23; 32; 40) configured to execute the predetermined functions;
a sensor (42) configured to detect that the subject introduction device (3) is located in the subject (1) via an opening (41) provided in an outer surface of a package (44) of the subject introduction device (3);
a cover (43) that covers the opening (41) with a covering material capable of reacting with a substance in the subject (1) when the subject introduction device (3) comes into contact with the substance located along a path of the subject introduction device (3), so as to expose the opening (41) with an advance of the reaction over time; and
a driving controller (34) configured to drive control the function executing unit (23; 32; 40) when the sensor (42) detects that the subject introduction device (3) is located in the subject (1),
**characterized in that** the sensor (42) is a humidity sensor and **in that** the cover (43) is formed with a material capable of melting in the substance in the subject (1), said substance being water or a predisposing material to be introduced in the stomach.

2. The subject introduction device (3) according to claim 1, wherein the cover (43) is configured to set an exposure time period of the opening (41) according to a thickness of the covering material at the opening (41).

3. The subject introduction device (3) according to claim 1, wherein the sensor (42) is configured to detect predetermined characteristics of the substance in the subject (1).

4. The subject introduction device (3) according to claim 1, wherein
the function executing unit (23) is a wireless transmitter (23) that includes a wireless actuating switch (23a) configured to actuate the wireless transmitter (23), and
the driving controller (34) is configured to turn on the wireless actuating switch (23a) when the sensor (42) detects that the subject introduction device (3) is located in the subject (1).

5. The subject introduction device (3) according to claim 4, further comprising a timer (26a; 32c) configured to count a predetermined time after the sensor (42) detects that the subject introduction device (3) is located in the subject (1), and further configured to switch the wireless actuating switch (23a) on when the timer (26a; 32c) counts up the predetermined time.

6. The subject introduction device (3) according to claim 5, wherein
the function executing unit (23) is a wireless transmitter (23) that includes a wireless power source changing unit (23b) configured to switch between a low power consumption mode and a normal power consumption mode of the wireless transmitter (23), and
the driving controller (34) is configured to send a change direction to the wireless power source changing unit (23b) to switch from the low power consumption mode to the normal power consumption mode when the sensor (42) detects that the subject introduction device (3) is located in the subject (1) or when the timer (26a; 32c) counts up the predetermined time.

7. The subject introduction device (3) according to claim 5, wherein the timer (26a) is provided into an imaging circuit (40).

8. The subject introduction device (3) according to claim 1, further comprising a frame rate changing unit (32b) configured to change between a first imaging process at a predetermined frame rate and a second imaging process at a frame rate higher than the predetermined frame rate, wherein
the driving controller (34) is configured to send a direction to the frame rate changing unit (32b) to perform image capturing at the frame rate of the first imaging process after a power source switch (34a) is on, and further configured to send a direction to perform image capturing at the frame rate of the second imaging process when a body determining unit (32a) determines that the subject introduction device (3) is located In the subject (1) or a timer (32c) counts up to the predetermined time.

9. A wireless in vivo information acquiring system comprising:
a subject introduction device (3) according to claim 1; and
a receiver (2) which is arranged out of the subject (1), and receives information obtained by the subject introduction device (3), via wireless communication, wherein
the receiver (2) includes
a wireless receiver (11) configured to receive the information transmitted from the wireless transmitter (23); and
a processor (12) configured to analyze the information received.

10. The wireless in vivo information acquiring system according to claim 9, wherein
the driving controller (34) is configured to turn on a wireless switch (23a) so as to actuate the wireless transmitter (23) when the sensor (42) detects that the subject introduction device (3) Is located in the subject (1).

## Patentansprüche

1. In eine Person einzuführende Vorrichtung (3) zum Einführen in eine Person (1), um vorbestimmte Funktionen in der Person (1) durchzuführen, die aufweist:
eine Funktionsausführungseinheit (23; 32; 40), die dazu ausgebildet ist, die vorbestimmten Funktionen auszuführen;
einen Sensor (42), der dazu ausgebildet ist, über eine Öffnung (41), die in einer Außenseite eines Gehäuses (44) der in eine Person einzuführenden Vorrichtung (3) vorgesehen ist, zu erfassen, dass sich die in eine Person einzuführende Vorrichtung (3) in der Person (1) befindet;
eine Abdeckung (43), die die Öffnung (41) mit einem Abdeckungsmaterial bedeckt, das mit einer Substanz in der Person (1) zu reagieren vermag, wenn die in eine Person einzuführende Vorrichtung (3) mit der Substanz in Kontakt kommt, die sich entlang eines Pfades der in eine Person einzuführenden Vorrichtung (3) befindet, um so die Öffnung (41) mit einem Fortschreiten der Reaktion im Laufe der Zeit freizulegen; und
eine Antriebssteuerung (34), die dazu ausgebildet ist, die Funktionsausführungseinheit (23; 32; 40) antriebsmäßig zu steuern, wenn der Sensor (42) erfasst, dass die in eine Person einzuführende Vorrichtung (3) sich in der Person (1) befindet,
**dadurch gekennzeichnet, dass** der Sensor (42) ein Feuchtigkeitssensor ist, und dass die Abdeckung (43) aus einem Material gebildet ist, das in der Substanz in der Person (1) zu schmelzen vermag, wobei die Substanz Wasser oder ein prädisponierendes, in den Magen einzuführendes Material ist.

2. In eine Person einzuführende Vorrichtung (3) nach Anspruch 1, wobei die Abdeckung (43) dazu ausgebildet ist, eine Freilegungszeitspanne der Öffnung (41) gemäß einer Stärke des Abdeckungsmaterials an der Öffnung (41) festzulegen.

3. In eine Person einzuführende Vorrichtung (3) nach Anspruch 1, wobei der Sensor (42) dazu ausgebildet ist, vorbestimmte Eigenschaften der Substanz in der Person (1) zu erfassen.

4. In eine Person einzuführende Vorrichtung (3) nach Anspruch 1, wobei die Funktionsausführungseinheit (23) ein Funksender (23) ist, der einen drahtlosen Auslöseschalter (23a) aufweist, der dazu ausgebildet ist, den Funksender (23) zu betätigen, und
die Antriebssteuerung (34) dazu ausgebildet ist, den drahtlosen Auslöseschalter (23a) anzuschalten, wenn der Sensor (42) erfasst, dass die in eine Person einzuführende Vorrichtung (3) sich in der Person (1) befindet.

5. In eine Person einzuführende Vorrichtung (3) nach Anspruch 4, die weiterhin einen Timer (26a; 32c) aufweist, der dazu ausgebildet ist, eine vorbestimmte Zeit zu zählen, nachdem der Sensor (42) erfasst, dass die in eine Person einzuführende Vorrichtung (3) sich in der Person (1) befindet, und ferner dazu ausgebildet ist, den drahtlosen Auslöseschalter (23a) anzuschalten, wenn der Timer (26a; 32c) die vorbestimmte Zeit hochzählt.

6. In eine Person einzuführende Vorrichtung (3) nach Anspruch 5, wobei
die Funktionsausführungseinheit (23) ein Funksender (23) ist, der eine drahtlose Energiequellenwechseleinheit (23b) aufweist, die dazu ausgebildet ist, zwischen einem Modus eines geringen Energieverbrauchs und einem Modes eines normalen Energieverbrauchs des Funksenders (23) zu schalten, und
die Antriebssteuerung (34) dazu ausgebildet ist, einen Richtungswechsel an die drahtlose Energiequellenwechseleinheit (23b) zu senden, um vom Modus des geringen Energieverbrauchs in den Modus des normalen Energieverbrauchs zu schalten, wenn der Sensor (42) erfasst, dass die in eine Person einzuführende Vorrichtung (3) sich in der Person (1) befindet, oder wenn der Timer (26a; 32c) die vorbestimmte Zeit hochzählt.

7. In eine Person einzuführende Vorrichtung (3) nach Anspruch 5, wobei der Timer (26a) in einer Bildgebungsschaltung (40) vorgesehen ist.

8. In eine Person einzuführende Vorrichtung (3) nach Anspruch 1, die weiterhin eine Frameraten-Wechseleinheit (32b) aufweist, die dazu ausgebildet ist, zwischen einem ersten Bildgebungsprozess mit einer vorbestimmten Framerate und einem zweiten Bildgebungsprozess mit einer höheren Framerate als die vorbestimmte Framerate zu wechseln, wobei
die Antriebssteuerung (34) dazu ausgebildet ist, eine Richtung an die Frameraten-Wechseleinheit (32b) zu senden, um eine Bilderfassung mit der Framerate des ersten Bildgebungsprozesses durchzuführen, nachdem ein Stromquellenschalter (34a) angeschaltet wurde, und ferner dazu ausgebildet ist, eine Richtung zu senden, um eine Bilderfassung mit der Framerate des zweiten Bildgebungsprozesses durchzuführen, wenn eine Körperbestimmungseinheit (32a) bestimmt, dass die in eine Person einzuführende Vorrichtung (3) sich in der Person (1) befindet, oder ein Timer (32c) die vorbestimmte Zeit hochzählt.

9. Drahtloses In-vivo-Informationserfassungssystem, das aufweist:
eine in eine Person einzuführende Vorrichtung (3) nach Anspruch 1; und
einen Empfänger (2), der außerhalb der Person (1) angeordnet ist und Informationen empfängt, die von der in eine Person einzuführende Vorrichtung (3) über Funkübertragung erhalten wurden, wobei
der Empfänger (2) aufweist
einen Funkempfänger (11), der dazu ausgebildet ist, die vom Funksender (23) übertragenen Informationen zu empfangen; und
einen Prozessor (12), der dazu ausgebildet ist, die empfangenen Informationen zu analysieren.

10. Drahtloses In-vivo-Informationserfassungssystem nach Anspruch 9, wobei
die Antriebssteuerung (34) dazu ausgebildet ist, einen drahtlosen Schalter (23s) anzuschalten, um so den Funksender (23) zu betätigen, wenn der Sensor (42) erfasst, dass sich die in eine Person einzuführende Vorrichtung (3) in der Person (1) befindet.

## Revendications

1. Dispositif d'introduction (3) dans un sujet pour insertion dans un sujet (1) pour exécuter des fonctions prédéterminées dans le sujet (1), comprenant :
une unité d'exécution de fonctions (23 ; 32 ; 40) configurée pour exécuter les fonctions prédéterminées ;
un capteur (42) configuré pour détecter que le dispositif d'introduction (3) dans un sujet est placé dans le sujet (1) via une ouverture (41) prévue dans une surface externe d'une enveloppe (44) du dispositif d'introduction (3) dans un sujet ;
un couvercle (43) qui couvre l'ouverture (41) avec un matériau de recouvrement capable de réagir avec une substance dans le sujet (1) lorsque le dispositif d'introduction (3) dans un sujet vient en contact avec la substance placée le long d'un trajet du dispositif d'introduction (3) dans un sujet, de façon à exposer l'ouverture (41) avec une avance de la réaction sur le temps ; et
un contrôleur de pilotage (34) configuré pour piloter l'unité d'exécution de fonctions (23 ; 32 ; 40) lorsque le capteur (42) détecte que le dispositif d'introduction (3) dans un sujet est placé dans le sujet (1),
**caractérisé en ce que** le capteur (42) est un capteur d'humidité et **en ce que** le couvercle (43) est formé avec un matériau capable de fondre dans la substance dans le sujet (1), ladite substance étant de l'eau ou un produit de préparation devant être introduit dans l'estomac.

2. Dispositif d'introduction (3) dans un sujet selon la revendication 1, dans lequel le couvercle (43) est configuré pour fixer une période de temps d'exposition de l'ouverture (41) conformément à une épaisseur du matériau de recouvrement au niveau de l'ouverture (41).

3. Dispositif d'introduction (3) dans un sujet selon la revendication 1, dans lequel le capteur (42) est configuré pour détecter des caractéristiques prédéterminées de la substance dans le sujet (1).

4. Dispositif d'introduction (3) dans un sujet selon la revendication 1, dans lequel
l'unité d'exécution de fonctions (23) est un émetteur sans fil (23) qui comprend un commutateur de mise en marche (23a) configuré pour actionner l'émetteur sans fil (23), et
le contrôleur de pilotage (34) est configuré pour activer le commutateur de mise en marche sans fil (23a) lorsque le capteur (42) détecte que le dispositif d'introduction (3) dans un sujet est placé dans le sujet (1).

5. Dispositif d'introduction (3) dans un sujet selon la revendication 4, comprenant en outre un compteur (26a ; 32c) configuré pour compter un temps prédéterminé après que le capteur (42) a détecté que le dispositif d'introduction (3) dans un sujet est placé dans le sujet (1), et en outre configuré pour activer le commutateur de mise en marche sans fil (23a) lorsque le compteur (26a ; 32c) compte le temps prédéterminé.

6. Dispositif d'introduction (3) dans un sujet selon la revendication 5, dans lequel
l'unité d'exécution de fonctions (23) est un émetteur sans fil (23) qui comprend une unité de changement de source d'énergie sans fil (23b) configurée pour commuter entre un mode à faible consommation d'énergie et un mode à consommation d'énergie normale de l'émetteur sans fil (23), et
le contrôleur de pilotage (34) est configuré pour envoyer une instruction de changement vers l'unité de changement de source d'énergie sans fil (23b) pour commuter du mode à faible consommation d'énergie au mode à consommation d'énergie normale lorsque le capteur (42) détecte que le dispositif d'introduction (3) dans un sujet est placé dans le sujet (1) ou lorsque le compteur (26a ; 32c) compte le temps prédéterminé.

7. Dispositif d'introduction (3) dans un sujet selon la revendication 5, dans lequel le compteur (26a) est prévu dans un circuit d'imagerie (40).

8. Dispositif d'introduction (3) dans un sujet selon la revendication 1, comprenant en outre une unité de changement de fréquence d'image (32b) qui est configurée pour effectuer un changement entre un premier processus d'imagerie à une fréquence d'image prédéterminée et un deuxième processus d'imagerie à une fréquence d'image supérieure à la fréquence d'image prédéterminée, dans lequel
le contrôleur de pilotage (34) est configurée pour envoyer une instruction à l'unité de changement de fréquence d'image (32b) pour réaliser une capture d'images à la fréquence d'image du premier processus d'imagerie après l'activation d'un commutateur de source d'énergie (34a), et en outre configuré pour envoyer une instruction pour réaliser une capture d'images à la fréquence d'image du deuxième processus d'imagerie lorsqu'une unité de détermination de corps (32a) détermine que le dispositif d'introduction (3) dans un sujet est placé dans le sujet (1) ou qu'un compteur (32c) compte jusqu'au temps prédéterminé.

9. Système d'acquisition d'informations in vivo sans fil comprenant :
un dispositif d'introduction (3) dans un sujet selon la revendication 1 ; et
un récepteur (2) qui est disposé à l'extérieur du sujet (1) et reçoit des informations obtenues par le dispositif d'introduction (3) dans un sujet, via communication sans fil, dans lequel
le récepteur (2) comprend
un récepteur sans fil (11) configuré pour recevoir les informations transmises depuis l'émetteur sans fil (23) ; et
un processeur (12) configuré pour analyser les informations reçues.

10. Système d'acquisition d'informations in vivo sans fil selon la revendication 9, dans lequel
le contrôleur de pilotage (34) est configuré pour activer un commutateur sans fil (23a) de façon à actionner l'émetteur sans fil (23) lorsque le capteur (42) détecte que le dispositif d'introduction (3) dans un sujet est placé dans le sujet (1).
